# EUROPEAN PATENT APPLICATION

(11) **EP 3 943 913 A1**
(43) Date of publication of application: **26.01.2022**
(21) Application number: 20773232.2
(22) Date of filing: 13.03.2020
(51) Int. Cl.: G01N 15/14, B01J 19/00, B03B 5/00, B03B 5/62, G01N 21/03, G01N 35/08, G01N 37/00

(54) **PARTICLE MEASURING DEVICE, PARTICLE SEPARATING AND MEASURING DEVICE, AND PARTICLE SEPARATING AND MEASURING APPARATUS**

(30) Priority: 20.03.2019 JP 2019052591
(71) Applicant: Kyocera Corporation, Kyoto-shi, Kyoto 612-8501 (JP)
(72) Inventor: YONETA,Masashi, Kyoto-shi, Kyoto 612-8501 (JP); MASUDA,Yuji, Kyoto-shi, Kyoto 612-8501 (JP)
(74) Representative: Viering, Jentschura & Partner mbB Patent- und Rechtsanwälte
(86) International application number: PCT/JP2020/011217
(87) International publication number: WO 2020/189572

(57) **Abstract**

A particle measuring device (3) has an upper surface having a first flow inlet (23) to receive a first fluid containing target particles to be measured, a first flow path (16) connected to the first flow inlet (23) to allow measurement of the target particles, and a third flow path (29) located upstream from and connected to a joint between the first flow path (16) and the first flow inlet (23) and having a smaller width than the first flow inlet (23). The first flow path (16) includes a first planar portion (27) having a greater width than the third flow path (29) and the first flow inlet (23), a width-increasing portion (16a) located downstream from and connected to the first planar portion (27), and a second planar portion (28) located downstream from and connected to the width-increasing portion (16a).

## Description

### FIELD

The present disclosure relates to a particle measuring device including flow paths for measuring particles contained in a liquid, and a particle separating and measuring device and a particle separating and measuring apparatus including the particle measuring device for separating and measuring target particles from multiple types of particles contained in a liquid.

### BACKGROUND

A known particle separating device separates and extracts particles from a liquid using a microfluidic structure (micro flow paths) several to several hundred micrometers wide and having a flow inlet and multiple flow outlets (refer to, for example, Japanese Patent Application Laid-Open No. 2012-76016). Such a particle separating device receives a liquid (e.g., blood) containing, for example, multiple types of particles (e.g., erythrocytes and leukocytes) through the flow inlet, separates target particles (e.g., leukocytes) from the liquid, and individually extracts the target particles and the other particles through the multiple flow outlets.

The separated and extracted target particles are then measured for, for example, their type, number, density, or optical properties.

### BRIEF SUMMARY

A particle measuring device according to an aspect of the present disclosure includes a flow path device being plate-like and having a plurality of flow paths inside. The flow path device has an upper surface having a first flow inlet to receive a first fluid containing target particles to be measured, and a second flow inlet to receive a second fluid free from the target particles. The plurality of flow paths include a first flow path connected to the first flow inlet to allow a flow of the first fluid and measurement of the target particles, a second flow path connected to the second flow inlet to allow a flow of the second fluid, and a third flow path located upstream from and connected to a joint between the first flow path and the first flow inlet in a planar direction and having a smaller width than the first flow inlet. The first flow path includes a first planar portion located at the joint with the first flow inlet and having a greater width than the third flow path and the first flow inlet, a width-increasing portion located downstream from and connected to the first planar portion and having a flow path width increasing downstream, and a second planar portion located downstream from and connected to the width-increasing portion and having a greater width than the first planar portion.

A particle separating and measuring device according to another aspect of the present disclosure includes a particle separating device being plate-like and having a pre-separation flow inlet to receive a fluid containing target particles to be separated, a main flow path connected to the pre-separation flow inlet, a plurality of branch flow paths connected to the main flow path, and a post-separation flow outlet to allow discharge of a first fluid containing the target particles after being separated, and the particle measuring device according to the above aspect including a first region receiving the particle separating device, and a second region defining a region to allow measurement of the target particles. The particle separating device having a lower surface having the post-separation flow outlet is on the particle measuring device having un upper surface having the first flow inlet in the first region, with the post-separation flow outlet facing and connecting to the first flow inlet.

A particle separating and measuring apparatus according to still another aspect of the present disclosure includes the particle separating and measuring device according to the above aspect, an optical sensor that emits light toward measurement portions of the first flow path and the second flow path in the particle separating and measuring device, and receives light passing through the measurement portions of the first flow path and the second flow path, and a controller that measures the target particles by comparing an intensity of the light passing through the measurement portion of the first flow path and received by the optical sensor with an intensity of the light passing through the measurement portion of the second flow path and received by the optical sensor.

### BRIEF DESCRIPTION OF DRAWINGS

FIG. 1 is a top view of an example particle separating and measuring device according to an embodiment of the present disclosure.
FIG. 2 is a cross-sectional view of the example particle separating and measuring device according to the embodiment of the present disclosure.
FIG. 3 is a plan view of an example particle separating device in the particle separating and measuring device according to the embodiment of the present disclosure.
FIG. 4 is a partial plan view of the example particle separating device in the particle separating and measuring device according to the embodiment of the present disclosure.
FIG. 5 is a partial cross-sectional view of the example particle separating and measuring device according to the embodiment of the present disclosure.
FIG. 6 is a partial cross-sectional view of an example particle separating and measuring device according to the embodiment of the present disclosure.
FIG. 7 is a partial cross-sectional view of an example particle separating and measuring device according to the embodiment of the present disclosure.
FIG. 8 is a plan view of an example particle measuring device according to the embodiment of the present disclosure.
FIG. 9 is a partial plan view of the example particle measuring device according to the embodiment of the present disclosure.
FIG. 10 is a cross-sectional view of the example particle measuring device in the particle separating and measuring device according to the embodiment of the present disclosure.
FIG. 11 is a cross-sectional view of an example particle separating and measuring apparatus including the particle separating and measuring device according to the embodiment of the present disclosure.
FIG. 12 is a block diagram of the particle separating and measuring apparatus according to the embodiment of the present disclosure, showing its example overall structure.
FIG. 13 is a plan view of an example particle measuring device according to the embodiment of the present disclosure.
FIG. 14 is a partial plan view of the example particle measuring device according to the embodiment of the present disclosure.

### DETAILED DESCRIPTION

A particle separating device for separating target particles in a liquid includes micro flow paths including a main flow path and multiple branch flow paths connected to the main flow path. The device receives a liquid specimen containing particles to be separated as well as multiple types of particles, and also receives a fluid for generating a pressing flow from the main flow path to the branch flow paths. The liquid containing the particles separated by the particle separating device then flows into a particle measuring device, where the liquid is introduced into flow paths in a measurement portion for measurement of, for example, the density of the particles. A particle separating and measuring device combines the particle separating device and the particle measuring device connected together to perform these tasks in a series of procedures.

The particle separating and measuring device may allow a liquid containing particles separated in the particle separating device to smoothly flow into the particle measuring device with reduced particle accumulation at the joint.

The particle measuring device according to one or more embodiments of the present disclosure includes a first flow inlet to receive a first fluid containing target particles to be measured, a first flow path connected to the first flow inlet to allow a flow of the first fluid for measuring the target particles, and a third flow path located upstream from and connected to a joint between the first flow path and the first flow inlet in a planar direction and having a smaller width than the first flow inlet. The first flow path for measuring the target particles includes a first planar portion located at the joint with the first flow inlet and having a greater width than the third flow path and the first flow inlet, a width-increasing portion located downstream from and connected to the first planar portion and having a flow path width increasing downstream, and a second planar portion located downstream from and connected to the width-increasing portion and having a greater width than the first planar portion. This structure reduces the likelihood that the target particles in the first fluid spread upstream along the inner wall of the flow path after flowing through the first flow inlet into the first planar portion. The first planar portion temporarily holds the target particles contained in the first fluid, and then allows the target particles to diffuse through the width-increasing portion into the second planar portion. This reduces unevenness of the particles for measurement in the second planar portion.

In the particle separating and measuring device and the particle separating and measuring apparatus according to one or more embodiments of the present disclosure, the particle separating device having a post-separation flow outlet in the lower surface is on the particle measuring device having the first flow inlet in the upper surface in a first region. The post-separation flow outlet faces and connects to the first flow inlet. Thus, the first fluid containing the target particles separated by the particle separating device flows into the first flow path through the first flow inlet in the particle measuring device without the unevenness of the target particles in the first flow path. This allows reliable and accurate measurement.

A particle measuring device, and a particle separating and measuring device and a particle separating and measuring apparatus including the particle measuring device according to one or more embodiments of the present disclosure will now be described with reference to the drawings. In one or more embodiments of the present disclosure, a Cartesian coordinate system (X, Y, Z) is defined for convenience, and the positive Z-direction is upward. However, any direction may be defined as upward or downward in one or more embodiments of the present disclosure. The embodiments below are examples of the present disclosure, and the present disclosure is not limited to the embodiments.

### Particle separating and measuring device

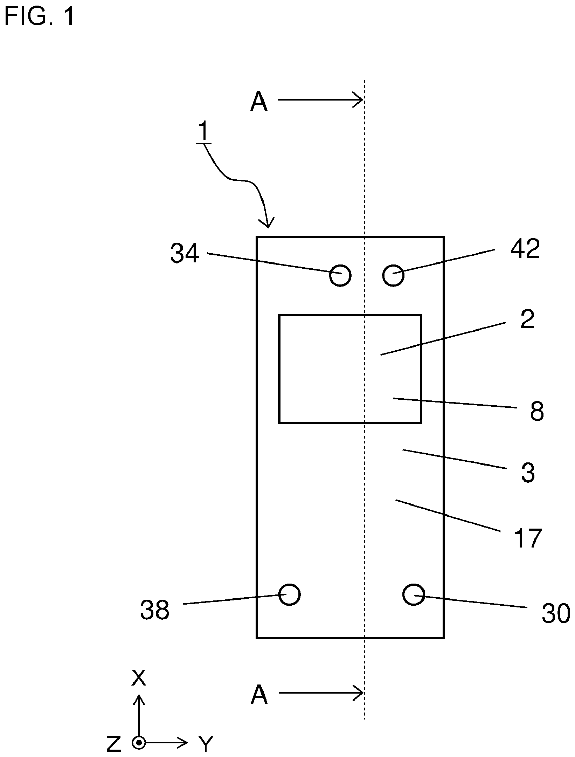

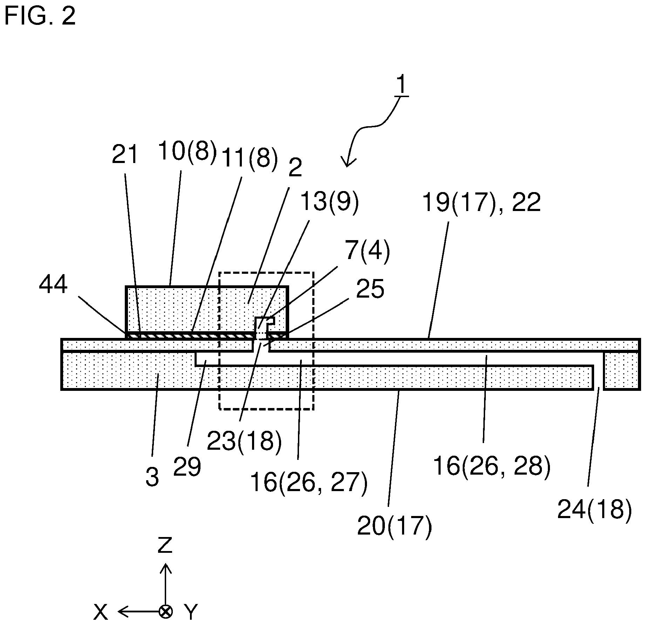

FIGs. 1 and 2 schematically show an example particle measuring device according to an embodiment of the present disclosure and an example particle separating and measuring device including the particle measuring device. FIG. 1 is a top view of a particle separating and measuring device 1. FIG. 2 is a cross-sectional view of the particle separating and measuring device 1 taken along line A-A shown in FIG. 1.

In the particle separating and measuring device 1, a fluid (specimen) containing target particles to be separated flows through a first flow path device 2 as a particle separating device. The first flow path device 2 thus separates and collects the target particles. The target particles (separated particles) then flow through a second flow path device 3 as a particle measuring device connected to the first flow path device 2. The second flow path device 3 thus allows measurement of the target particles. For example, the particle separating and measuring device 1 separates and collects leukocytes as a target component from blood to allow measurement of the number of leukocytes.

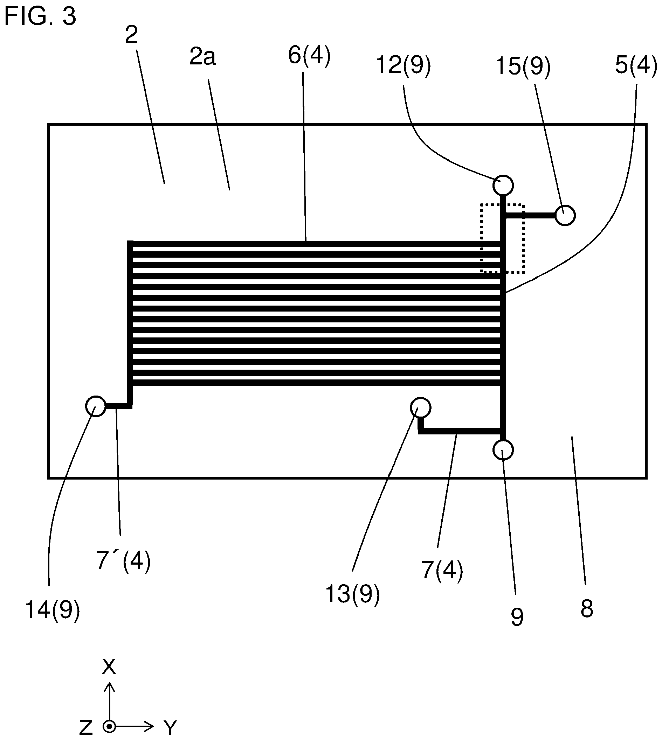

FIG. 3 schematically shows the example first flow path device 2 as a particle separating device. FIG. 3 is a plan view of the first flow path device 2 in top perspective.

### Particle separating device (first flow path device)

The first flow path device 2 is a particle separating device for separating and collecting target particles from a liquid (specimen) containing multiple types of particles including the target particles to be separated. The first flow path device 2 has a pre-separation flow inlet 12 to receive a fluid containing target particles to be separated, a main flow path 5 connected to the pre-separation flow inlet 12, multiple branch flow paths 6 connected to the main flow path 5, and a post-separation flow outlet 13 to allow discharge of the first fluid containing the target particles after being separated.

The first flow path device 2 is substantially plate-like. More specifically, the flow path device 2 includes a plate-like base 2a having a separating flow path 4 inside. The separating flow path 4 includes the straight main flow path 5 and the multiple branch flow paths 6 connected to and branching from the main flow path 5. In the first flow path device 2 according to the embodiment of the present disclosure, a specimen (e.g., blood) flows into the main flow path 5. Then, particles (second particles, or for example, erythrocytes) different from target particles (first particles, or for example, leukocytes) flow from the main flow path 5 into the branch flow paths 6. Thus, the target particles (the first particles) in the specimen are separated. The second particles in the specimen may be separated by flowing into the branch flow paths 6.

The branch flow paths 6 branch from the main flow path 5 to receive the second particles. However, particles flowing into the branch flow paths 6 are not limited to the second particles. Particles different from the second particles (e.g., third particles) may flow into the branch flow paths 6.

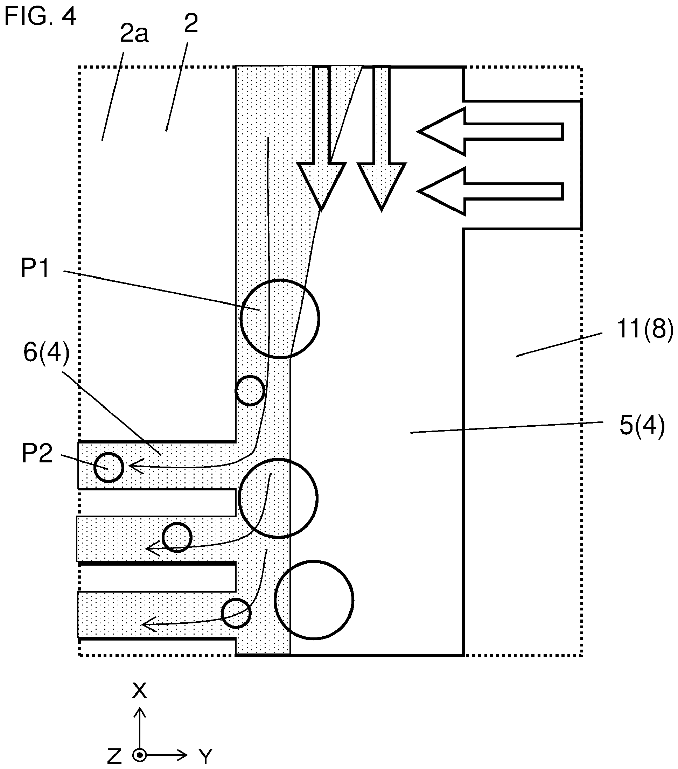

FIG. 4 schematically shows the main flow path 5 and the branch flow paths 6 separating the first particles and the second particles. FIG. 4 is an enlarged plan view of the region enclosed by the broken line in FIG. 3. In FIG. 4, the larger circles indicate first particles PI, and the smaller circles indicate second particles P2. The hatched arrows in X-direction indicate the main flow, and the white arrows in Y-direction indicate a pressing flow (described later). The hatched region in the figure indicates a lead-in flow (described later).

The separating flow path 4 in the embodiment of the present disclosure includes the single main flow path 5 and the multiple branch flow paths 6 orthogonal to and connected to the main flow path 5 along the main flow path 5. The first flow path device 2 generates a lead-in flow in the main flow path 5 flowing from the main flow path 5 to the branch flow paths 6 by adjusting, for example, the cross-sectional areas and the lengths of the main flow path 5 and the branch flow paths 6 and the flow velocity of the specimen. The first flow path device 2 generates a pressing flow in the separating flow path 4 for pressing the specimen through the main flow path 5 into the branch flow paths 6. As shown in FIG. 4, the branch flow paths 6 that receive the lead-in flow each have a width smaller than the size of the first particles P1 (target particles) flowing in the specimen and larger than the size of the second particles P2 (other particles). Thus, the second particles P2 are led into the branch flow paths 6. The lead-in flow is pressed by the pressing flow and moves along the main flow path 5 adjacent to the branch flow paths 6. The lead-in flow has a width larger than the distance between the edge of the main flow path 5 and the center of gravity of the second particles P2 flowing in the specimen and smaller than the distance between the edge and the center of gravity of the first particles P1. Thus, the second particles P2 are effectively led into the branch flow paths 6. This allows the first particles P1 to be separated as target particles in the specimen and collected with the flow through the main flow path 5. This also allows the second particles P2 to be separated in the specimen and collected with the flow through the branch flow paths 6.

The first flow path device 2 according to the embodiment of the present disclosure may be used to separate erythrocytes and leukocytes in blood as a specimen. Erythrocytes in blood have, for example, a size of 6 to 8 µm and the center of gravity 3 to 4 µm away from the edge. Leukocytes have, for example, a size of 10 to 30 µm and the center of gravity 5 to 15 µm away from the edge. In this case, the main flow path 5 may have, for example, a cross-sectional area of 300 to 1000 µm² and a length of 0.5 to 20 mm. The main flow path 5 may have, for example, a cross section having an area within the above range, a width of about 30 µm, and a height of about 20 µm. The branch flow paths 6 may each have, for example, a cross-sectional area of 100 to 500 µm² and a length of 3 to 25 mm. The branch flow paths 6 may have, for example, a cross section having an area within the above range, a width of about 15 µm, and a height of about 20 µm. The flow velocity in the separating flow path 4 may be 0.2 to 5 m/s, for example. With these dimensions, the lead-in flow may have a width of, for example, 2 to 15 µm, allowing effective separating of erythrocytes and leukocytes in blood.

The target particles may be any of various extracellular vesicles, instead of leukocytes or erythrocytes. Examples of extracellular vesicles include exosomes (30 to 200 nm), microvesicles (200 to 1000 nm), and large oncosomes (1 to 10 µm). The target particles may be inorganic matter or target fine particles in a fluid, such as a suspension containing fine powder. In either case, the separating flow path 4 may have a shape and dimensions designed as appropriate for, for example, the size of the target particles to be separated.

The first flow path device 2 has multiple first openings 9 in one or both of the upper surface and the lower surface of the base 2a. At least two of the first openings 9 are flow inlets for receiving a specimen and a fluid to flow into the main flow path 5. The flow inlets include the pre-separation flow inlet 12 and a pressing-flow inlet 15. The pre-separation flow inlet 12 receives a specimen as a fluid containing target particles (e.g., the first particles PI) to be separated, and supplies the specimen to the main flow path 5. The pressing-flow inlet 15 is orthogonally connected to a portion of the main flow path 5 upstream from and opposite to the multiple branch flow paths 6 with respect to the main flow path 5. The pressing-flow inlet 15 receives a fluid for generating a pressing flow.

The first opening 9 as the pre-separation flow inlet 12 may be circular and have a dimension of, for example, 1 to 3 mm. The flow paths in the separating flow path 4 may have the same height. The pre-separation flow inlet 12 may have a depth, for example, corresponding to the distance from the opening in the upper surface of the base 2a to the bottom surface of the main flow path 5.

The first opening 9 as the pressing-flow inlet 15 may be circular and have a dimension of, for example, 1 to 3 mm. A flow path for a pressing flow may have the same height as the other flow paths in the separating flow path 4. The pressing-flow inlet 15 may have a depth, for example, corresponding to the distance from the opening in the upper surface of the base 2a to the bottom surface of the main flow path 5.

The separating flow path 4 further includes a collection flow path 7 connected to the main flow path 5. The collection flow path 7 may be used to collect the separated first particles P1. In the separating flow path 4 in the embodiment of the present disclosure, the first particles P1 are collected in the collection flow path 7 using a pressing flow.

The separating flow path 4 may also include a disposal flow path 7' connected to the multiple branch flow paths 6. The second particles P2 separated by the branch flow paths 6 may be collected or disposed through the disposal flow path 7'. In some embodiments, the multiple branch flow paths 6 collect the second particles P2, which are then collected in the single disposal flow path 7' connected to the branch flow paths 6. In this case, the fluid containing the first particles P1 may flow from the main flow path 5 to the collection flow path 7 and may then be disposed.

The first flow path device 2 includes the plate-like base 2a. The plate-like base 2a has the separating flow path 4 inside. The first flow path device 2 has a pair of first upper and lower surfaces 8 at the top and bottom in the thickness direction (Z-direction). The separating flow path 4 has the multiple first openings 9 in one or both of the pair of first upper and lower surfaces 8.

For convenience, one of the pair of first upper and lower surfaces 8 is referred to as a first upper surface 10 and the other as a first lower surface 11 in the embodiment of the present disclosure. The first upper surface 10 of the pair of first upper and lower surfaces 8 is located in the positive Z-direction, and the first lower surface 11 is located in the negative Z-direction. In the embodiment of the present disclosure, at least one of the multiple first openings 9 is located in the first lower surface 11.

The multiple first openings 9 include at least the pre-separation flow inlet 12, the post-separation flow outlet 13, and at least one disposal flow outlet 14. The pre-separation flow inlet 12 receives a specimen to flow into the main flow path 5. The post-separation flow outlet 13 discharges the first fluid containing the separated first particles P1 (target particles) for collection from the collection flow path 7. The disposal flow outlet 14 disposes, for collection, the components of the specimen excluding the first particles P1. In the embodiment of the present disclosure, the first openings 9 include the pressing-flow inlet 15 for receiving a fluid for generating a pressing flow that presses the specimen toward the branch flow paths 6. In the embodiment of the present disclosure, the disposal flow outlet 14 is connected to the main flow path 5 and the disposal flow path 7'. The fluid disposed through the disposal flow outlet 14 is collected through a through-hole 14' in the second flow path device 3 (described later).

The first flow path device 2 according to the embodiment of the present disclosure is rectangular as viewed from above. The first upper and lower surfaces 8 are flat. The first flow path device 2 may not be rectangular as viewed from above. The first upper and lower surfaces 8 may not be flat. The first upper and lower surfaces 8 (the first upper surface 10 and the first lower surface 11) may have different shapes.

The first flow path device 2 is formed from, for example, polydimethylsiloxane (PDMS) or polymethyl methacrylate or acrylic resin (PMMA). The first flow path device 2 may have a thickness of, for example, 1 to 5 mm. The first flow path device 2 may be, for example, rectangular as viewed from above with short sides of 10 to 20 mm and long sides of 10 to 30 mm. The first flow path device 2 is formed by, for example, preparing two substrates, forming grooves for the separating flow path 4 on one of the substrates, and placing the other substrate to cover the grooves and bonding the substrates together to complete the base 2a having the separating flow path 4 inside.

### Particle measuring device (second flow path device)

The second flow path device 3 as a particle measuring device is used to measure target particles separated and collected by the first flow path device 2. The second flow path device 3, together with the first flow path device 2, forms a particle separating and measuring device. The second flow path device 3 has an upper surface including a first region 21 receiving the first flow path device 2 and a second region 22 defining a region to allow measurement of target particles. The second flow path device 3 also has a first flow inlet 23 to receive the first fluid, and a second flow inlet to receive a second fluid free from the target particles (described later). The second flow path device 3 also has a first flow path 16 located in the second region 22 and connected to the first flow inlet 23 to allow a flow of the first fluid, and a second flow path (described later) located in the second region 22 and connected to the second flow inlet to allow a flow of the second fluid. The second flow path device 3 is substantially plate-like, similarly to the first flow path device 2.

As shown in FIG. 2, the second flow path device 3 has the first flow path 16 connected to the separating flow path 4 in the first flow path device 2. The second flow path device 3 is translucent. In the second flow path device 3, the first fluid containing target particles separated and collected by the first flow path device 2 flows through the first flow path 16, in which the target particles are measured with an optical sensor (described later). More specifically, the target particles are measured by measuring the intensity of light passing through the first fluid containing the target particles through the first flow path 16.

The second flow path device 3 includes a plate-like base having flow paths inside. The plate-like base has the first flow path 16 inside. The second flow path device 3 has a pair of second upper and lower surfaces 17 at the top and bottom in the thickness direction (Z-direction). The first flow path 16 has multiple second openings 18 in one or both of the pair of second upper and lower surfaces 17.

For convenience, one of the pair of second upper and lower surfaces 17 is referred to as a second upper surface 19 and the other as a second lower surface 20 in the embodiment of the present disclosure. The second upper surface 19 of the pair of second upper and lower surfaces 17 is located in the positive Z-direction, and the second lower surface 20 is located in the negative Z-direction.

The second flow path device 3 according to the embodiment of the present disclosure is rectangular as viewed from above. The second upper and lower surfaces 17 are flat. The second flow path device 3 may not be rectangular as viewed from above. The second upper and lower surfaces 17 may not be flat. The second upper and lower surfaces 17 (the second upper surface 19 and the second lower surface 20) may have different shapes.

The second flow path device 3 is formed from, for example, acrylic resin (PMMA) or cycloolefin polymer (COP). The second flow path device 3 may have a thickness of, for example, 0.5 to 5 mm. The second flow path device 3 may be, for example, rectangular as viewed from above with short sides of 20 to 40 mm and long sides of 20 to 80 mm. The second flow path device 3 is formed by, for example, preparing two substrates, forming a groove for the first flow path 16 on one of the substrates, and placing the other substrate to cover the groove and bonding the substrates together to complete the base having the first flow path 16 inside.

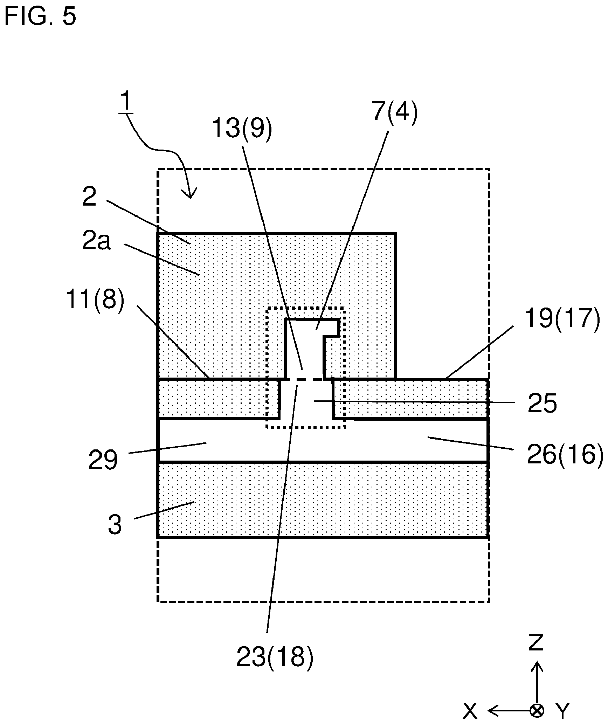

FIG. 5 is a partial schematic view of the example particle separating and measuring device 1 including the first flow path device 2 as a particle separating device and the second flow path device 3 as a particle measuring device. FIG. 5 is an enlarged cross-sectional view of the region enclosed by the broken line in FIG. 2.

In the second flow path device 3 according to the embodiment of the present disclosure, at least one of the multiple second openings 18 is located in the second upper surface 19. The second upper surface 19 includes the first region 21 receiving the first flow path device 2 with the first lower surface 11 on the first region 21. One of the first openings 9 located in the first lower surface 11 as the post-separation flow outlet 13 faces and connects to one of the second openings 18 located in the second upper surface 19 as the first flow inlet 23. In the particle separating and measuring device 1 according to the embodiment of the present disclosure, the flow path in the first flow path device 2 is directly connected to the flow path in the second flow path device 3. This allows target particles in a specimen to be separated, collected, and measured sequentially, improving processing efficiency. The particle separating and measuring device 1, in which the first flow path device 2 and second flow path device 3 are stacked in the thickness direction, is downsized.

The second flow path device 3 according to the embodiment of the present disclosure has the second upper surface 19 including the first region 21 receiving the first flow path device 2, and the second region 22 defining the region to allow measurement of target particles. As viewed from above, the first flow path 16 in the second flow path device 3 extends over the first region 21 to the second region 22, whereas the first flow path device 2 extends in the first region 21 in the second flow path device 3 alone. The first flow path 16 is thus located in the second region 22 without overlapping the first flow path device 2. Thus, the second region 22 is used for measuring particles with the first flow path 16 used as a measurement flow path.

The particle separating and measuring device 1 may include a light reflector in the second region 22 as described later.

The first flow path device 2 and the second flow path device 3 may be formed from different materials. In the embodiment of the present disclosure, for example, the first flow path device 2 is formed from PDMS, and the second flow path device 3 is formed from COP.

As shown in the embodiment of the present disclosure, the first flow path device 2 is above the second flow path device 3. More specifically, the first flow path device 2 is located on the first region 21 in the second upper surface 19 of the second flow path device 3. Thus, the first fluid containing the target particles separated and collected by the first flow path device 2 efficiently flows into the second flow path device 3 using the gravity. This reduces accumulation of the first fluid containing the target particles in the flow path, for example, at a joint between the first flow path device 2 and the second flow path device 3.

The present disclosure does not exclude embodiments in which the first flow path device 2 is located on the second lower surface 20 of the second flow path device 3.

The multiple second openings 18 include the first flow inlet 23 and a first flow outlet 24. The first flow inlet 23 receives the first fluid containing the separated target particles to flow into the first flow path 16. The first flow outlet 24 discharges the first fluid from the first flow path 16 for collection. The first flow inlet 23 has an opening located in the second upper surface 19. The first flow inlet 23 faces and connects to the post-separation flow outlet 13 in the first flow path device 2. The first flow outlet 24 is located in the second lower surface 20. Thus, the first fluid smoothly enters the first flow inlet 23 from the first flow path device 2 with the gravity, thus facilitating collection of the first fluid through the first flow outlet 24.

### Connection structure between first flow path device and second flow path device

The first flow path device 2 is placed on the first region 21 in the second upper surface 19 of the second flow path device 3. The post-separation flow outlet 13 in the first flow path device 2 faces and connects to the first flow inlet 23 in the second flow path device 3. In the embodiment of the present disclosure, the second opening 18 of the first flow outlet 24 is larger than the first opening 9 of the post-separation flow outlet 13, as shown in FIG. 5. This reduces accumulation of the first fluid at the joint between the first flow path device 2 and the second flow path device 3. The post-separation flow outlet 13 may have an opening with a dimension of, for example, 0.5 to 3 mm, and more specifically, about 2 mm. The first flow outlet 24 may have an opening with a dimension of, for example, 1.5 to 6 mm, and more specifically, about 2.5 mm.

The post-separation flow outlet 13 and the first flow inlet 23 basically have circular openings, but the openings may have other shapes depending on the characteristics of the target particles and the first fluid. For example, they may be elliptic or rectangular, or specifically, square, rectangular, or rhombic. For elliptic openings, the minor axes may align with the direction in which any other flow paths are located near the openings, and the major axes may align with the direction in which sufficient space is provided around the openings. This flow path can have less interference with other flow paths. For rhombic openings, the first fluid can be easily controlled to have different flow velocities between the central portion and the peripheral portion of each opening. This may allow flow control at the joint.

The post-separation flow outlet 13 and the first flow inlet 23 are basically concentrically face each other, but may face each other with their centers being out of alignment. When the post-separation flow outlet 13 has its center offset downstream along the first flow path 16 relative to the center of the first flow inlet 23, the first fluid tends to more easily flow downstream along the first flow path 16 due to, for example, the flow of the second fluid (described later).

The first flow path 16 includes the vertical portion 25 connected to the first flow inlet 23 (the second opening 18) and extending in the thickness direction, and a planar portion 26 connected to the vertical portion 25 and extending in a direction in a plane over the second region 22. The first flow path 16 including the vertical portion 25 reduces accumulation of the first fluid at the joint with the separating flow path 4. The first flow path 16 can hold the first fluid in the planar portion 26 for measurement of particles, thus allowing reliable measurement.

The vertical portion 25 may have a width of, for example, 1.5 to 4 mm. The planar portion 26 may have a width of, for example, 1.5 to 6 mm. The vertical portion 25 may have a length of, for example, 0.5 to 1 mm. The planar portion 26 may have a height of, for example, 0.5 to 2 mm.

In the example shown in FIG. 2, a sheet member 44 is placed between the first flow path device 2 and the second flow path device 3. However, the sheet member 44 is optional and may not be included as in the example shown in FIG. 5. The first flow path device 2 and the second flow path device 3 may be directly connected to each other with a silane coupling agent applied to one or both of the first lower surface 11 of the first flow path device 2 and the second upper surface 19 of the second flow path device 3.

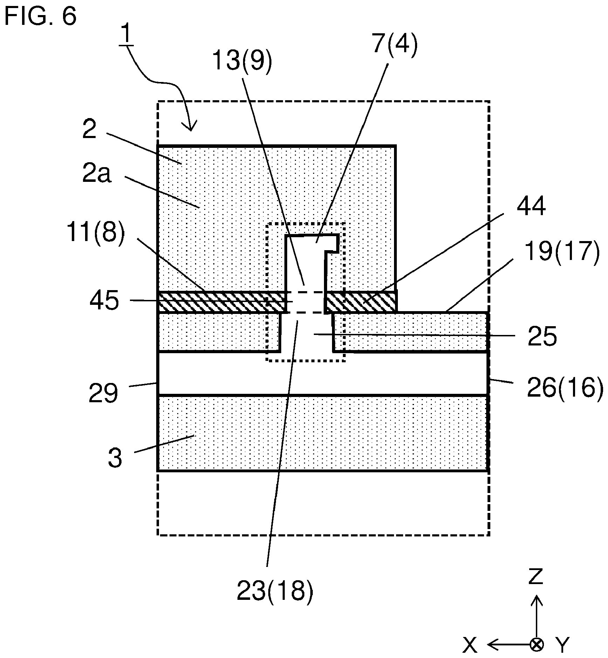

As in a cross-sectional view of FIG. 6 similar to FIG. 5, the sheet member 44 may be placed between the first lower surface 11 of the first flow path device 2 and the second upper surface 19 of the second flow path device 3, as in the example shown in FIG. 2. In other words, the particle separating and measuring device 1 may include the sheet member 44 between the first flow path device 2 and the second flow path device 3. More specifically, the first flow path device 2 may be placed on the second flow path device 3 with the sheet member 44 in between, and the post-separation flow outlet 13 and the first flow inlet 23 may connect to each other with a through-hole 45 in the sheet member 44. The through-hole 45 in the sheet member 44 may have an opening with substantially the same dimension as or larger than the opening of the post-separation flow outlet 13. The openings with substantially the same dimension may include openings having dimensional differences within manufacturing tolerances.

The through-hole 45 is smaller than the opening of the first flow inlet 23. Similarly to the example shown in FIG. 5, the structure allows the target particles to efficiently flow from the post-separation flow outlet 13 through the through-hole 45 into the first flow inlet 23 at the center of the first flow inlet 23 and further flow through the vertical portion 25 effectively into the first flow path 16 at the center. The fluid flowing through the first flow path 16 allows uniform dispersion of the target particles in the fluid. This allows accurate measurement.

The through-hole 45 in the sheet member 44 may have a uniform dimension in the vertical direction. In some embodiments, the through-hole 45 may be flared downward. The through-hole 45 flared downstream may increase the distribution of target particles flowing through the through-hole 45 into the first flow inlet 23.

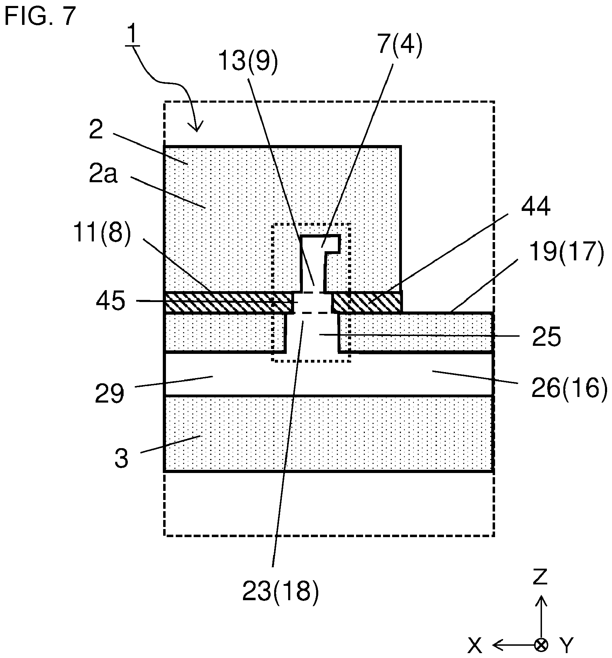

For the first flow path device 2 and the second flow path device 3 formed from materials that are difficult to adhere to each other, the sheet member 44 as an intermediate layer can firmly bond the devices, thus stably forming the particle separating and measuring device 1. As in a cross-sectional view of FIG. 7 similar to FIG. 6, the through-hole 45 between the post-separation flow outlet 13 and the first flow inlet 23 may have the opening with an appropriate dimension that falls between the dimensions of the openings of the post-separation flow outlet 13 and the first flow inlet 23. This effectively prevents accumulation of the first fluid and the target particles at the joint between the first flow path device 2 and the second flow path device 3.

The sheet member 44 reduces leakage of, for example, the first fluid from the bonding surfaces of the first flow path device 2 and the second flow path device 3. The sheet member 44 also serves as an intermediate layer for bonding materials that are difficult to adhere to each other. The sheet member 44 may be of a material such as silicone or PDMS. The sheet member 44 also accommodates any deformation of the first lower surface 11 and the second upper surface 19 as bonding surfaces. The sheet member 44 may have multiple through-holes as appropriate, in addition to the through-hole 45 between the post-separation flow outlet 13 and the first flow inlet 23. The multiple through-holes, including the through-hole 45, face multiple first openings 9 and second openings 18. The fluid thus flows from the first flow path device 2 through these through-holes to the second flow path device 3.

The sheet member 44 may have a thickness of, for example, about 0.5 to 3 mm. The sheet member 44 having a thickness of about 2 mm can sufficiently accommodate any deformation of the bonding surfaces, and also shorten the distance between the post-separation flow outlet 13 and the first flow inlet 23. The sheet member 44 with such a thickness can also reduce cracks or other damage when the first flow path device 2 and the second flow path device 3 are bonded together.

The sheet member 44 may have any appropriate dimensions (area) large enough for adhesion around the through-hole 45 and smaller than or equal to the dimensions of the first lower surface 11 of the first flow path device 2. The sheet member 44 may not be a single sheet, and may be a combination of multiple sheets with predetermined shapes and dimensions.

The first flow path device 2 and the second flow path device 3 according to the embodiment of the present disclosure may be directly connected to the sheet member 44, or may be connected with an adhesive applied to the upper and lower surfaces of the sheet member 44. The adhesive may be, for example, a photo-curable resin curable with ultraviolet light or a thermoplastic resin.

In the particle separating and measuring device 1 according to the embodiment of the present disclosure, the through-hole 45 in the sheet member 44 may have the opening larger than the opening of the post-separation flow outlet 13 and smaller than the opening of the first flow inlet 23, as in a cross-sectional view of FIG. 7 similar to FIG. 6. For example, the through-hole 45 may have the opening with a dimension of 2 to 2.5 mm when the post-separation flow outlet 13 has the opening with a dimension of 1.5 to 2 mm and the first flow inlet 23 has the opening with a dimension of 2.5 to 3 mm. The through-hole 45 may have a combination of dimensions within the above ranges to have the opening larger than the opening of the post-separation flow outlet 13 and smaller than the opening of the first flow inlet 23. The structure allows the target particles to efficiently flow and spread from the post-separation flow outlet 13 through the through-hole 45 into the first flow inlet 23 and further flow through the vertical portion 25 effectively into the center of the first flow path 16. The fluid flowing through the first flow path 16 allows uniform dispersion of the target particles in the fluid. This allows accurate measurement.

The particle separating and measuring device 1 according to the embodiment of the present disclosure may include, between the first flow path device 2 and the second flow path device 3, the sheet member 44 having a higher hardness than the first flow path device 2 and a lower hardness than the second flow path device 3. This allows the flow path in the softer first flow path device 2 to maintain its shape on the flat, harder base sheet member 44 between the first flow path device 2 and the sheet member 44. The sheet member 44 is tightly as well as firmly bonded to the second flow path device 3, serving as the harder base, between the second flow path device 3 and the sheet member 44. The bonding surfaces of the first flow path device 2 and the sheet member 44 may have substantially the same surface roughness. The bonding surfaces of the sheet member 44 and the second flow path device 3 may have substantially the same surface roughness. More specifically, these bonding surfaces may have an arithmetic mean roughness Ra of about 0.005 to 0.05 µm.

To evaluate the hardness of the components, International Rubber Hardness Degrees (IRHD) are typically used for rubber molded products, and Rockwell hardness for resin molded products. The hardness of the components herein can be measured with IRHD for relative evaluation. For example, the first flow path device 2 may have a hardness of at least 30 and lower than 80 under IRHD, the sheet member 44 may have a hardness of about 80 under IRHD, and the second flow path device 3 may have a hardness of higher than 80 under IRHD. For example, the first flow path device 2 may be formed from PDMS, the sheet member 44 from silicone, and the second flow path device 3 from COP or PMMA to achieve a combination of the above hardness. More specifically, PDMS is about 30 under IRHD, silicone is about 80 under IRHD, and COP exceeds 80 under IRHD (about 50 in Rockwell hardness). These materials may be used to achieve a combination of the above hardness.

The hardness can be measured by pressing an unsharp indenter into an object to be measured with a predetermined force, and measuring and quantifying its deformation. For durometer hardness, the force for pressing the indenter is applied with a spring. For IRHD, the force for pressing the indenter is applied with, for example, a weight for applying a constant load. Durometer hardness, which can be measured with a simpler instrument, is more common and may be used.

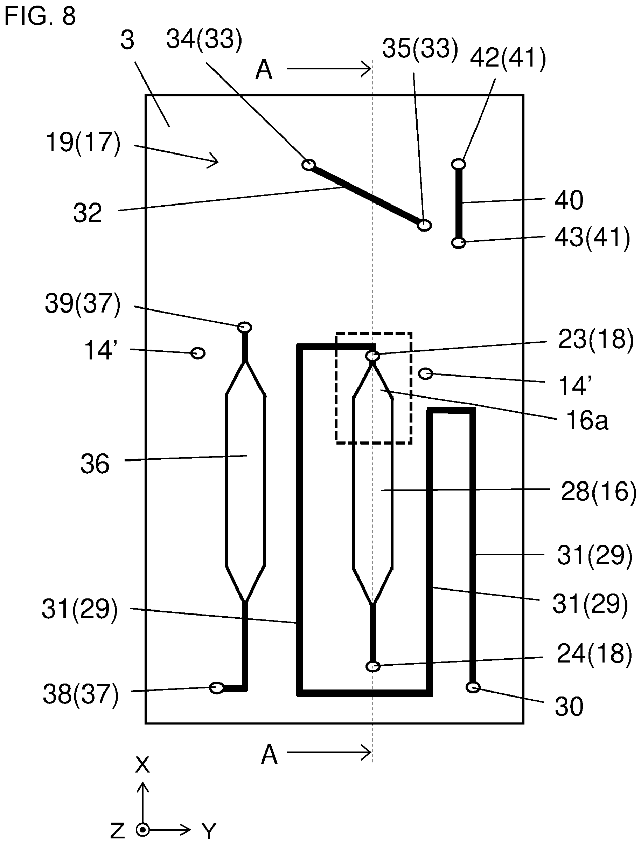

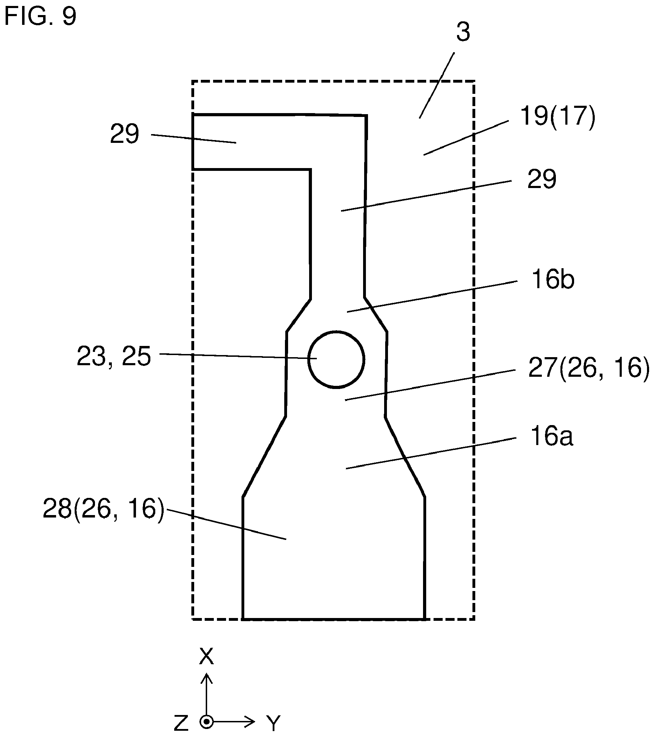

FIGs. 8 and 9 schematically show the example second flow path device 3 included in the particle separating and measuring device 1. FIG. 8 is a plan view of the second flow path device 3 in top perspective. FIG. 9 is an enlarged plan view of the region enclosed by the broken line in FIG. 8. Line A-A in FIG. 8 is at the same position as line A-A in FIG. 1.

The planar portion 26 in the first flow path 16 includes a first planar portion 27 connected to the vertical portion 25 and having a greater width than the vertical portion 25, a width-increasing portion 16a located downstream from and connected to the first planar portion 27 and having a flow path width increasing downstream, and a second planar portion 28 located downstream from and connected to the width-increasing portion 16a and having a greater width than the first planar portion 27. The second flow path device 3 includes a third flow path 29 located upstream from and connected to the joint between the first flow path 16 and the first flow inlet 23 in the planar direction. The third flow path 29 has a greater width than the first planar portion 27. More specifically, the second flow path device 3 includes the first planar portion 27 and the width-increasing portion 16a between the first flow inlet 23 and the second planar portion 28 located in the second region 22 and used as the measurement portion in the first flow path 16. The first planar portion 27 has a greater width than the vertical portion 25. The width-increasing portion 16a has a flow path width increasing downstream along the flow of the first fluid. The third flow path 29 is located upstream from and connected to the first planar portion 27. The first planar portion 27 has a greater width than the third flow path 29 and the first flow inlet 23.

The first planar portion 27 is connected to the vertical portion 25 and has a greater width than the vertical portion 25. This structure reduces accumulation of the first fluid at the joint between the planar portion 26 and the vertical portion 25. The third flow path 29 is located upstream from and connected to the first planar portion 27 in the first flow path 16, and has a smaller width than the first flow inlet 23. This structure reduces the likelihood that the target particles in the first fluid spread upstream along the inner wall of the flow path after flowing through the first flow inlet 23 into the first planar portion 27. The first planar portion 27 in the first flow path 16 has a greater width than the first flow inlet 23, and has a greater width than the third flow path 29 having a smaller width than the first flow inlet 23. Thus, the first planar portion 27 temporarily holds the target particles contained in the first fluid, and then allows the target particles to flow through the width-increasing portion 16a into the second planar portion 28. The width-increasing portion 16a causes a flow of the first fluid to spread in the width direction to disperse the target particles contained in the first fluid. This reduces unevenness of the particles for measurement in the second planar portion 28. The third flow path 29 receives, for example, a gas for forcing out the fluid accumulating in the planar portion 26 downstream. This reduces fluid accumulation in the first flow path 16 and allows repeated, reliable measurement of particles.

The first planar portion 27 may have a width of, for example, 0.7 to 3 mm. The second planar portion 28 may have a width of, for example, 1 to 5 mm. The second planar portion 28 may have a width of, for example, 2 to 10 times the width of the first planar portion 27. In the embodiment of the present disclosure, the width-increasing portion 16a has a width gradually increasing at the joint between the first planar portion 27 and the second planar portion 28. In other words, the width-increasing portion 16a is flared downstream along the flow path in the width direction. The flared portion widens toward the end at 20 to 40° on each side of the centerline across the width of the planar portion 26 (the first planar portion 27 and the second planar portion 28). The flared portion may have a length of, for example, about 3 to 5 mm.

The width-increasing portion 16a may widen in a curved or stepwise manner, rather than widening gradually in a straight manner. The width-increasing portion 16a connecting the first planar portion 27 to the second planar portion 28 may have a flow path width increasing stepwise, for example, from 1 mm to 2.5 mm, and from 2.5 mm to 5 mm. The width-increasing portion 16a having such a sharply increasing flow path width allows the second planar portion 28 to have a flow path width twice or more that of the first planar portion 27. This allows a vortex to occur in the first fluid flowing through these portions, facilitating agitation and mixing of the target particles contained in the first fluid.

As shown in FIG. 9, a second width-increasing portion 16b may connect the third flow path 29 and the first planar portion 27. The second width-increasing portion 16b has a flow path width increasing from the third flow path 29 toward the first planar portion 27. However, the flow path width may vary stepwise between the third flow path 29 and the first planar portion 27. The second width-increasing portion 16b reduces the likelihood that the target particles in the first fluid spread upstream along the inner wall of the flow path after flowing through the first flow inlet 23 into the first planar portion 27. The second width-increasing portion 16b also allows a fluid (e.g., saline free from the target particles or gas) from the third flow path 29 to smoothly push target particles from the first planar portion 27 into the second planar portion 28. This reduces particle accumulation in the first planar portion 27.

The third flow path 29 may have a width of, for example, about 0.5 to 1 mm. In the embodiment of the present disclosure, the second width-increasing portion 16b has a width gradually increasing at the joint between the third flow path 29 and the first planar portion 27. In other words, the second width-increasing portion 16b is flared downstream along the flow path in the width direction. The flared portion widens toward the end at 25 to 50° on each side of the centerline across the width of the third flow path 29 and the first planar portion 27. For leukocytes targeted, the flared portion may widen at, for example, about 27° on each side and about 53° as a whole. The flared portion may have a length of, for example, about 2 to 4 mm.

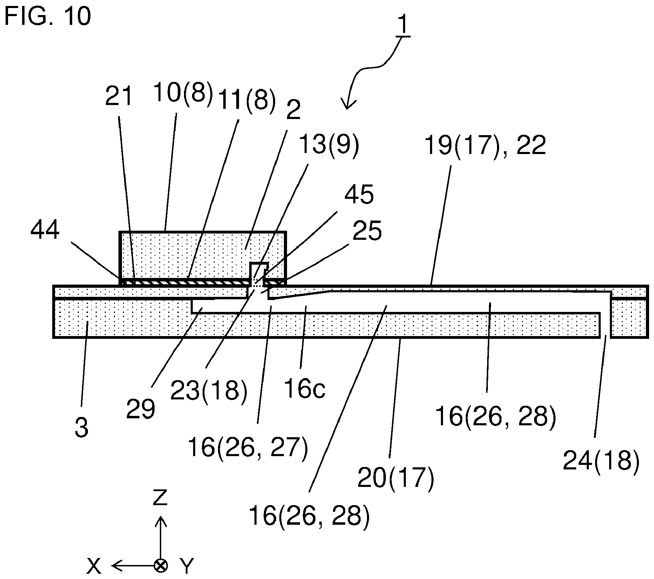

The second planar portion 28 may have a greater height than the first planar portion 27. As in a cross-sectional view of FIG. 10 similar to FIG. 2, the second flow path device 3 may include a height-increasing portion 16c between the first flow inlet 23 and the second planar portion 28 located in the second region 22 and used as the measurement portion in the first flow path 16. The height-increasing portion 16c has a flow path height increasing downstream along the flow of the first fluid. The height-increasing portion 16c causes a flow of the first fluid to spread in the height direction to disperse the target particles contained in the first fluid, reducing unevenness of the target particles for measurement. The flow path has a height increasing over a relatively short distance and thus allows a vortex to occur in the flow of fluid, facilitating agitation of the target particles. This facilitates the diffusion of the separated target particles (e.g., the first particles PI).

The first planar portion 27 may have a height of, for example, 0.2 to 1 mm. The second planar portion 28 may have a height of, for example, 1 to 5 mm. In the embodiment of the present disclosure, the height-increasing portion 16c has a height gradually increasing at the joint between the first planar portion 27 and the second planar portion 28. In other words, the height-increasing portion 16c is flared downstream along the flow path in the height direction. For example, the first planar portion 27 may have a height of 0.5 mm, and the second planar portion 28 may have a height of 1 mm, with the flared portion being flared at about 45°.

For the planar portion 26 in the first flow path 16 including both the width-increasing portion 16a and the height-increasing portion 16c, the height-increasing portion 16c may be immediately upstream from the width-increasing portion 16a. The width-increasing portion 16a and height-increasing portion 16b may be closest possible to each other. In the flow path having the width greater than the height, the height-increasing portion may be upstream from the width-increasing portion. This structure allows the fluid to be vertically agitated in the height-increasing portion with a narrow width and then laterally agitated with the increasing width. This allows more uniform agitation. A width-increasing portion located upstream can reduce the effects of agitation in the height direction.

As shown in FIGs. 8 and 9, the third flow path 29 has one end connected to the first flow path 16 in the second flow path device 3 according to the embodiment of the present disclosure. The third flow path 29 has the other end being a third opening 30 located in the pair of second upper and lower surfaces 17. More specifically, the third flow path 29 has the third opening 30 located in one of the pair of second upper and lower surfaces 17 (the second upper surface 19 in the embodiment of the present disclosure). The third opening 30 receives a displacement fluid (e.g., gas) for forcing another fluid out of the second planar portion 28 in the first flow path 16.

As shown in FIG. 8, the third flow path 29 may have a portion connected to the first flow path 16 and at least partially extending along the extension of the planar portion 26 (the second planar portion 28) in the first flow path 16. As shown in FIG. 8, the third flow path 29 may include multiple straight portions 31 extending in a predetermined direction and arranged in a direction intersecting the direction. The third flow path 29 including the multiple straight portions 31 reduces the fluid flowing back from the first flow path 16 and leaking from the third opening 30.

The first openings 9 as the pre-separation flow inlet 12 and the post-separation flow outlet 13 may be in the same surface (the first lower surface 11 in the embodiment of the present disclosure). In this case, a specimen flows into the first flow path device 2 from below (in the negative Z-direction). In this structure, the second particles P2 having a greater specific gravity than the first particles P1 sink and are thus easily separated.

As shown FIG. 8, the second flow path device 3 may further include a fourth flow path 32 different from the first flow path 16 and the third flow path 29. The fourth flow path 32 may have multiple fourth openings 33 located in one or both of the pair of second upper and lower surfaces 17. The fourth flow path 32 allows a specimen to flow before target particles are separated in the specimen. The fourth flow path 32 in the second flow path device 3 allows the specimen to flow to reduce foreign matter before entering the first flow path device 2.

The multiple fourth openings 33 include a fourth flow inlet 34 and a fourth flow outlet 35. The fourth flow inlet 34 is an opening for receiving the specimen to flow into the fourth flow path 32. The fourth flow outlet 35 is an opening for discharging the specimen from the fourth flow path 32. The fourth flow inlet 34 is open to receive the specimen from outside. The fourth flow outlet 35 is connected to the pre-separation flow inlet 12 in the first flow path device 2.

The fourth flow inlet 34 and the fourth flow outlet 35 may be in the second upper surface 19. In this case, an operator can handle the device from above for, for example, connecting the device with an external component to supply a specimen. In the embodiment of the present disclosure, the fourth flow inlet 34 is in the same surface as the first flow outlet 24. In the embodiment of the present disclosure, the fourth flow outlet 35 is also in the same surface as the first flow outlet 24. The fourth flow inlet 34 is in the same surface as the third opening 30.

As shown in FIG. 8, the second flow path device 3 includes a second flow path 36 different from the first flow path 16, the third flow path 29, and the fourth flow path 32. The first flow path 16 is used for the first fluid containing the target particles separated and collected by the first flow path device 2, whereas the second flow path 36 is used for the second fluid free from the target particles. For example, the second flow path 36 is used for the second fluid for comparison or calibration for measuring the first fluid. The second fluid may be the same fluid as the first fluid but excluding the target particles, or may be a different fluid. For every measurement of the target particles, the first flow path 16 and the second flow path 36 may sequentially undergo measurement to determine the difference in light intensity between them. The difference can be used to estimate the number of target particles. The results are less susceptible to deterioration of the optical sensor.

The second flow path 36 has multiple fifth openings 37 located in the pair of second upper and lower surfaces 17. The fifth openings 37 include a second flow inlet 38 and a second flow outlet 39. The second flow inlet 38 is an opening for receiving the second fluid to flow into the second flow path 36. The second flow outlet 39 is an opening for discharging the second fluid from the second flow path 36. The second flow path 36 includes a measurement portion similarly shaped to the second planar portion 28 in the first flow path 16.

The second flow inlet 38 as one of the multiple fifth openings 37 is in the same surface as the third opening 30. In this case, an operator can handle the device on the same surface from above for, for example, supplying and discharging the second fluid. The second flow outlet 39 may be in the second lower surface 20.

The second flow path device 3 may further include a sixth flow path 40 different from the first flow path 16, the third flow path 29, the fourth flow path 32, and the second flow path 36. The sixth flow path 40 has multiple sixth openings 41 in one or both of the pair of second upper and lower surfaces 17. The multiple sixth openings 41 include a sixth flow inlet 42 and a sixth flow outlet 43. The sixth flow inlet 42 is an opening for receiving a fluid for generating a pressing flow to flow into the sixth flow path 40. The sixth flow outlet 43 is an opening for discharging the fluid for generating a pressing flow from the sixth flow path 40. The sixth flow inlet 42 is located to receive the fluid. The sixth flow outlet 43 is connected to the pressing-flow inlet 15 in the first flow path device 2.

The third flow path 29, the fourth flow path 32, the second flow path 36, and the sixth flow path 40 may be formed in the same manner as the first flow path 16.

### Particle separating apparatus

A particle separating apparatus in the particle separating and measuring apparatus according to the embodiment of the present disclosure will now be described. The particle separating apparatus according to the embodiment of the present disclosure includes the first flow path device 2 as a particle separating device, a first pump for pumping a specimen into the pre-separation flow inlet 12, and a second pump for pumping a fluid into the pressing-flow inlet 15. The particle separating device is the first flow path device 2 described above. The first flow path device 2 has the pre-separation flow inlet 12 connected to the first pump with, for example, a first tube. The first pump delivers a specimen, which then flows through the first tube into the pre-separation flow inlet 12 in the first flow path device 2. The first flow path device 2 has the pressing-flow inlet 15 connected to the second pump with, for example, a second tube. The second pump delivers a fluid, which flows through the second tube into the pressing-flow inlet 15 in the first flow path device 2. This structure allows target particles (e.g., the first particles PI) to be separated and collected from the specimen through the main flow path 5 and the multiple branch flow paths 6, as described above.

The first and second pumps may be any of a variety of known pumps that can pump a fluid. The first pump may be capable of pumping a small amount of fluid (e.g., blood) containing particles into the pre-separation flow inlet 12 in the first flow path device 2 at a constant flow velocity. The second pump may be capable of pumping a fluid for generating a pressing flow (e.g., phosphate buffered saline, or PBS) into the pressing-flow inlet 15 in the first flow path device 2 at an appropriate flow rate, flow velocity, and pressure. The first and second pumps may be, for example, syringe pumps. Other pumps such as electroosmotic pumps, peristaltic pumps, and gas pumps may also be used.

The first and second tubes may be formed from any of a variety of known materials in accordance with the fluid to be used. For example, silicone tubes may be used for blood as the specimen and PBS as the fluid. These tubes are optional and may be eliminated when, for example, the first flow path device 2 is connected to the first and second pumps directly or with adapters.

### Particle separating and measuring apparatus

A particle separating and measuring apparatus according to the embodiment of the present disclosure will now be described. The apparatus includes the particle separating and measuring device according to the embodiment of the present disclosure including the particle measuring device according to the embodiment of the present disclosure.

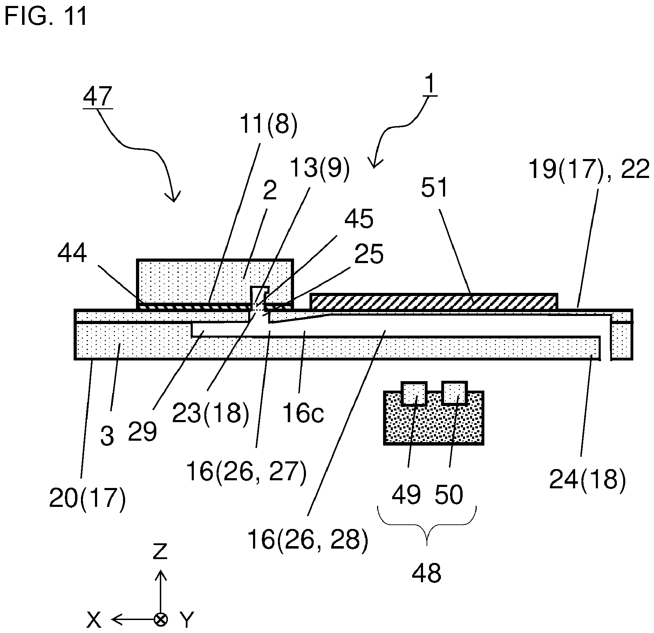

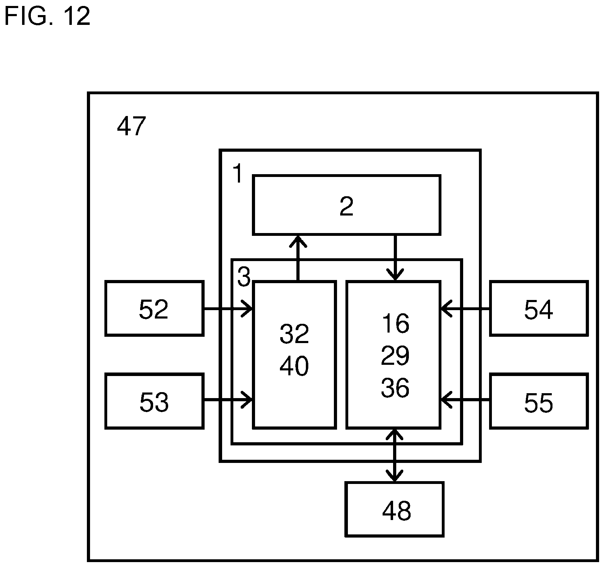

FIGs. 11 and 12 schematically show a particle separating and measuring apparatus 47. FIG. 11 is a cross-sectional view of the particle separating and measuring apparatus 47 as viewed from the same viewpoint as FIGs. 2 and 10. Some reference numerals are the same as those in FIGs. 2 and 10 and thus are not described. FIG. 12 is a block diagram of the particle separating and measuring apparatus 47, showing its example overall structure.

The particle separating and measuring apparatus 47 includes the particle separating and measuring device 1 and an optical sensor 48. The optical sensor 48 includes a light-emitting element 49 and a light receiving element 50. The first flow path device 2 in the particle separating and measuring device 1 separates intended target particles (e.g., the first particles PI) in the specimen. The target particles then flow into the first flow path 16 (the second planar portion 28) in the second flow path device 3 in the particle separating and measuring device 1. The optical sensor 48 emits light with the light-emitting element 49 toward the target particles, and receives, with the light receiving element 50, light passing through the first flow path 16 (the second planar portion 28) for measurement of the particles. More specifically, the light passing through the first flow path 16 is scattered, reflected, or absorbed by the particles (the first particles PI) in the first fluid and is thus attenuated in intensity. A calibration curve is predefined to show the relationship between the specimen having a known number of particles and the corresponding attenuation of light. The particles in the specimen can be measured by comparing the attenuation of received light measured by the particle separating and measuring apparatus 47 with the calibration curve.

The particle separating and measuring apparatus 47 according to the embodiment of the present disclosure includes the particle separating and measuring device 1 according to the embodiment of the present disclosure described above, the optical sensor 48, and a controller. The optical sensor 48 emits light toward the measurement portions in the first flow path 16 and the second flow path 36 in the particle separating and measuring device 1, and receives light passing through the measurement portions in the first flow path 16 and the second flow path 36. The controller measures target particles by comparing the intensity of the light passing through the measurement portion in the first flow path 16 and received by the optical sensor 48 with the intensity of the light passing through the measurement portion in the second flow path 36 and received by the optical sensor 48.

The light-emitting element 49 may be, for example, a light-emitting diode (LED). The light receiving element 50 may be, for example, a photodiode (PD). For example, the light receiving element 50 is a PD formed on the upper surface of a semiconductor substrate and having regions of one conductivity type and another conductivity type. The light-emitting element 49 is an LED including multiple semiconductor layers stacked on the semiconductor substrate.

The particle separating and measuring device 1 in the particle separating and measuring apparatus 47 according to the embodiment of the present disclosure includes a mirror 51 located in the second region 22 in the second upper surface 19 of the second flow path device 3. The optical sensor 48 has the light-emitting element 49 and the light receiving element 50 located adjacent to the second lower surface 20 of the second flow path device 3. Thus, light emitted from the light-emitting element 49 in the optical sensor 48 passes through the first flow path 16 (the second planar portion 28), is reflected by the mirror 51, and is then received by the light receiving element 50 in the optical sensor 48. The mirror 51 may be formed from, for example, aluminum or gold. The mirror 51 may be formed by, for example, depositing a metal foil with vapor deposition or sputtering.

The particle separating and measuring apparatus 47 further includes a first supply unit 52 for supplying a specimen, a second supply unit 53 for supplying a fluid for generating a pressing flow, a third supply unit 54 for supplying a displacement fluid, and a fourth supply unit 55 for supplying the second fluid as a calibration fluid. The first to fourth supply units 52 to 55 are connected to the particle separating and measuring device 1. The first supply unit 52 is connected to the fourth flow inlet 34. The second supply unit 53 is connected to the sixth flow inlet 42. The third supply unit 54 is connected to the third opening 30. The fourth supply unit 55 is connected to the second flow inlet 38. The particle separating and measuring apparatus 47 includes a controller (not shown) for controlling the first supply unit 52, the second supply unit 53, the third supply unit 54, the fourth supply unit 55, and the optical sensor 48.

The particle separating and measuring apparatus 47 according to the embodiment of the present disclosure includes the particle separating and measuring device 1 according to the embodiment of the present disclosure. Thus, the particle separating and measuring apparatus 47 separates target particles in a specimen for accurate and reliable measurement.

The present disclosure is not limited to the above embodiments, but may be changed and modified variously without departing from the spirit and scope of the present disclosure.

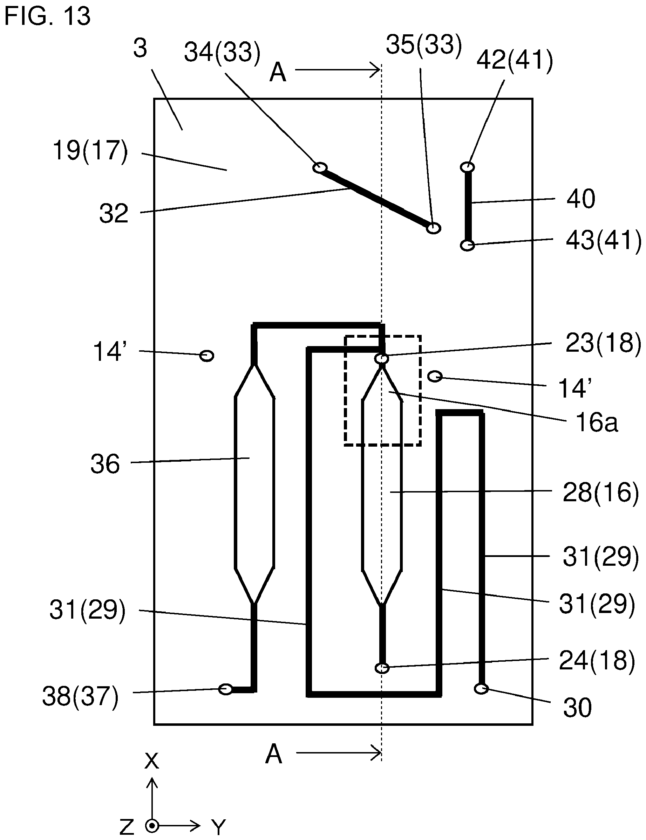

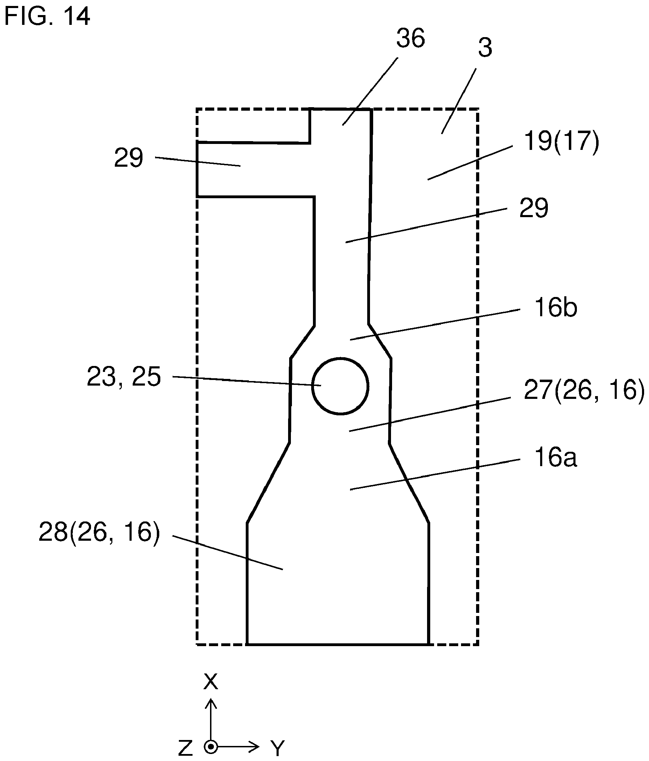

In the above embodiments, the second flow path 36 has one end being the second flow outlet 39. In some embodiments, the second flow path 36 may have one end connected to the first flow path 16 as shown in FIGs. 13 and 14. This structure allows the second fluid in the second flow path 36 to be injected into the first flow path 16 to reduce the density of target particles (e.g., leukocytes) contained in the first fluid in the first flow path 16. FIGs. 13 and 14 are similar to FIGs. 8 and 9 as viewed from a similar viewpoint, and are not described in detail.

In the above embodiments, the second flow path device 3 includes the second flow path 36 and the sixth flow path 40. In some embodiments, the second flow path 36 may serve as the sixth flow path 40. More specifically, the second flow path 36 and the sixth flow path 40 may be formed as a single flow path and connected to the separating flow path 4 (the pressing-flow inlet 15).

### Reference Signs List

- 1: particle separating and measuring device
- 2: particle separating device (first flow path device)
- 3: particle measuring device (second flow path device)
- 4: separating flow path
- 5: main flow path
- 6: branch flow path
- 7: collection flow path
- 12: pre-separation flow inlet
- 13: post-separation flow outlet
- 16: first flow path
- 16a: width-increasing portion
- 16b: second width-increasing portion
- 16c: height-increasing portion
- 21: first region
- 22: second region
- 23: first flow inlet
- 25: vertical portion
- 26: planar portion
- 27: first planar portion
- 28: second planar portion
- 29: third flow path
- 36: second flow path
- 38: second flow inlet
- 44: sheet member
- 45: through-hole
- 47: particle separating and measuring apparatus
- 48: optical sensor

## Claims

1. A particle measuring device comprising:
a flow path device being plate-like and having a plurality of flow paths inside, the flow path device having an upper surface having
a first flow inlet to receive a first fluid containing target particles to be measured, and a second flow inlet to receive a second fluid free from the target particles,
the plurality of flow paths including
a first flow path connected to the first flow inlet to allow a flow of the first fluid and measurement of the target particles,
a second flow path connected to the second flow inlet to allow a flow of the second fluid, and
a third flow path located upstream from and connected to a joint between the first flow path and the first flow inlet in a planar direction and having a smaller width than the first flow inlet,
wherein the first flow path includes
a first planar portion located at the joint with the first flow inlet and having a greater width than the third flow path and the first flow inlet,
a width-increasing portion located downstream from and connected to the first planar portion and having a flow path width increasing downstream, and
a second planar portion located downstream from and connected to the width-increasing portion and having a greater width than the first planar portion.

2. The particle measuring device according to claim 1, further comprising:
a second width-increasing portion between the third flow path and the first planar portion, the second width-increasing portion having a flow path width increasing from the third flow path toward the first planar portion.

3. A particle separating and measuring device comprising:
a particle separating device being plate-like and having
a pre-separation flow inlet to receive a fluid containing target particles to be separated,
a main flow path connected to the pre-separation flow inlet,
a plurality of branch flow paths connected to the main flow path, and
a post-separation flow outlet to allow discharge of a first fluid containing the target particles after being separated; and
the particle measuring device according to claim 1 including
a first region receiving the particle separating device, and
a second region defining a region to allow measurement of the target particles,
wherein the particle separating device having a lower surface having the post-separation flow outlet is on the particle measuring device having the upper surface having the first flow inlet in the first region, with the post-separation flow outlet facing and connecting to the first flow inlet.

4. The particle separating and measuring device according to claim 3, wherein
the particle separating device is on the particle measuring device with a sheet member in between, and the post-separation flow outlet and the first flow inlet connect to each other with a through-hole in the sheet member.

5. The particle separating and measuring device according to claim 4, wherein
the sheet member has a higher hardness than the particle separating device and a lower hardness than the particle measuring device.

6. A particle separating and measuring apparatus comprising:
the particle separating and measuring device according to any one of claims 3 to 5;
an optical sensor configured to emit light toward the first flow path and the second flow path in the particle separating and measuring device, and receive light passing through the first flow path and the second flow path; and
a controller configured to measure the target particles by comparing an intensity of the light passing through the first flow path and received by the optical sensor with an intensity of the light passing through the second flow path and received by the optical sensor.
